# EUROPEAN PATENT SPECIFICATION

(11) **EP 3 034 069 B1**
(45) Date of publication and mention of the grant of the patent: **16.09.2020**
(21) Application number: 15200393.5
(22) Date of filing: 16.12.2015
(51) Int. Cl.: A61P 1/12, A61P 17/00, A61P 19/02, A61P 37/04, A61P 39/00, A61K 9/00, A61K 47/02, A61K 35/744

(54) **HYGIENIC POWDER FOR USE IN THE FARROWING AREA OF SOWS**
HYGIENISCHES PULVER ZUR VERWENDUNG IM ABFERKELBEREICH VON SAUEN
POUDRE HYGIÉNIQUE DESTINÉE À ÊTRE UTILISÉE DANS LA ZONE DE MISE BAS DE TRUIES

(30) Priority: 18.12.2014 EP 14198915
(43) Date of publication of application: 22.06.2016
(73) Proprietor: DSM IP Assets B.V., 6411 TE Heerlen (NL)
(72) Inventor: DE VRIENDT, Dirk, 4303 Kaiseraugst (CH)
(74) Representative: Schwander, Kuno

(56) References cited:
- EP-A1- 1 574 128
- WO-A1-2013/067185
- CN-A- 101 703 072
- US-A1- 2007 166 295
- Anonymous: "Lactobacillus Serum - The Unconventional Farmer", , 18 August 2014 (2014-08-18), XP055191842, Retrieved from the Internet: URL:http://wayback.archive.org/web/2014081 8200115/http://theunconventionalfarmer.com /recipes/lactobacillus-serum/ [retrieved on 2015-05-28]
- Anonymous: "Eurosec", , 25 February 2014 (2014-02-25), pages 1-1, XP055191724, Heerlen Retrieved from the Internet: URL:http://www.fontanka.nl/_pages/en_euros ec.html [retrieved on 2015-05-26]
- "Eurosec Litter conditioner", , 7 October 2013 (2013-10-07), XP055191727, Heerlen Retrieved from the Internet: URL:http://www.fontanka.nl/_documents/Euro sec_ENG.pdf [retrieved on 2015-05-26]

## Description

### TECHNICAL FIELD

The present invention relates to hygienic powders as defined by the claims for use in the farrowing area of sows. The invention also relates to the use of such powders for preventing diseases and reducing mortality in piglets.

More specifically, the present invention relates to a drying & disinfectant powder composition for reducing heat loss and the risk of neonatal diarrhea in newborn piglets and for reducing of the overall use of antibiotics in pig farming.

### BACKGROUND ART

As competition in the pig-farming industry increases, priority has been put on economical and efficient production. As a result, infection of the intestines, respiratory diseases and the like can easily occur in pigs raised under stressful conditions.

High mortality rates of newborn piglets and pig diseases reduce farm productivity and generate big economic losses for pig farmers.

Piglets receive immune antibodies from mother pigs in lactation period so that the risk of infection is low. However, the antibodies disappear after weaning and, therefore, diseases can be developed easily.

Conventionally, drugs have been used as additional measures against diseases. As a result, many drug-resistant bacteria have been developed. Furthermore, by administrating antibiotics, toxins can be released from resistant bacteria proliferated in the intestines to rapidly increase the number of troubles so that pigs may be subjected to over-prescription of medications despite the intentions. CN 101 703 072 discloses a hygienic dry powder comprising kaolin as absorbent and borneo camphor as environment conditioning agent for sterilizing the environment of a pig house and prevention of infection of piglets, reduction of humidity and promotion of quick drying of piglets, improvement of immunity as well as lowering diarrhea rate of them.

In addition it is well known that the first days of newborn piglets are very critical and crucial for the survival rate, health status, and immune development of piglets. The cerebral cortex of newborn piglets doesn't develop well, the ability of adjusting body temperature via the nervous system is poor, the energy stored in the body is less, and it is difficult for them to survive if not taking colostrum timely because the blood glucose suffers from fast decrease in a cold environment. The normal body temperature is 39°C for piglets. The environmental temperature needs to be 30 - 32°C at birth. If the environmental temperature is lower, the body temperature of piglets will start drop and will rise after dropping to a certain temperature range. For newborn piglets, the range of drop in body temperature and the time needed by temperature recovery depend on the environmental temperature. The lower the environmental temperature is, the greater the range of drop in body temperature is and the longer the time needed by temperature recovery is. As studied, if newborn piglets stay in a 13 - 24°C environment, their body temperature can drop by 1.7 - 7.2°C one hour after birth. Particularly, the body temperature will drop more quickly due to evaporation of amniotic fluid if their body isn't dry 20 minutes after born. In addition, the earlier piglets take colostrum, the more quickly the body temperature recovers. If newborn piglets live in a 18 - 24°C environment, their body temperature will recover to normal after two days.

Moreover, other factors which influence survival rate are diseases resulting from infections with pathogenic microorganisms such as Escherichia coli. Young piglets infected with pathogenic Escherichia coli have a low resistance, so the mortality is relatively high.

### SUMMARY OF THE INVENTION

It is therefore the object of the present invention to provide a new concept that improves health and performance in piglets, in particular in newborn piglets, especially during stress conditions in the first days of life and when high performance are demanded.

Another object of the present invention is to provide a composition which is suitable for preventing diseases and enhancing the immune system in piglets, so as to prevent death due to stressful raising conditions and pathogenic diseases.

It has been surprisingly found that a powder composition of a mixture of natural minerals and at least one probiotic used in synergy and in combination exhibits, in totally unexpected manner, the effects sought by the present invention of improving health, boosting the immune system and growth of piglets as well as of reducing mortality of newborn piglets.

In the present invention, the hygienic powder composition has disinfectant properties and contains by weight ratio at least one lactic acid probiotic formulation (0.25% - 5%), kaolin (40% - 70%) and a mixture (30% - 60%) of conventional additives, such as flavorings, absorbing agents, drying agents and fillers.

In particular, the powder composition has the following properties (amongst others):
- It is dust-freer and easy to disperse
- It has strong moisture-absorbing properties
- It supports transfer of beneficial gut flora from the sow to the newborn piglet

The powder composition according to the invention has the following functions:
- It ensures that newborn piglets dry faster an reach the nipples early after birth guaranteeing
   ▪ earlier intake of colostrum which provides them with basic nutritional substances and antibodies essential at the beginning of their lives
   ▪ reduced temperature loss and improved vitality of piglets
   ▪ fewer arthritis cases and infections of the umbilical cord.
- It has a positive effect on the skin flora of piglets, reducing the risk of skin disorders, e.g. greasy pigs disorders
- Further to the colonization of the probiotic in the environment, multiplication of pathogenic germs such as E.coli is reduced
- The use of the powder composition stimulates microbial colonization of beneficial gut flora, which provides extra protection against neonatal diarrhea
- Better weight gain from the beginning
- Reduced mortality among piglets

The powder composition is intended to be used as follows:
- For newborn piglets. The composition is dispersed at least once a day, preferably twice a day on the floor of the farrowing (parturition) area. It can also be powdered onto the skin of the piglets and optionally onto the skin of the sow and onto the udder. The composition is applied at the day of parturition and at least at the 2, preferably at the 3 following days, optionally for the first 10 days of life.
- Optionally, for piglets during lactation, the powder is sprayed once a day into the piglet nursery box until weaning to repress bacterial reproduction in this small environment to decrease incidence of bacterial diarrhea while absorbing ammonia, hydrogen sulfide and other harmful gases to give a suitable living environment to piglets.

### DISCLOSURE OF THE INVENTION

The inventors have focused on the use of lactic acid bacteria that have been isolated and selected for their probiotic properties.

It has been observed that the death of piglets due to the risks as mentioned above is remarkably decreased when probiotics of the genera defined above are combined with kaolin and applied in form of a dust-free powder composition to the farrowing area of newborn piglets.

As mentioned above, the powder composition contains by weight ratio at least one probiotic formulation (0.25% - 5%), kaolin (40% - 70%) and a mixture of conventional additives (30% - 60%), such as flavorings, absorbing agents, drying agents and fillers, wherein the at least one probiotic strain formulation and kaolin are the main ingredients of the present invention.

The complete powder compositions according to the invention are in dried, dust-free form. These powders may be produced by ways known in the art provided that where micro-organism activity is desired, care is taken to ensure survival of the micro-organism. Apart from the bacteria strains, the powder compositions contain the probiotic microorganism in a form which ensures the required stability and properties of the microbe. For example the microbe is granulated and dehydrated on a bed composed of carbohydrates (for example lactose or saccharose) and subsequently sieved and optionally coated (for example with cellulose derivatives and resins).

The at least one probiotic strain is preferably a lactic acid bacteria of the genera Lactobacillus, Enterococcus and Bifidobacterium. The probiotic strain may be selected from the group consisting of Lactobacillus reuteri (for example CNCM I-2448), Lactobacillus reuteri (for example CNCM 1-2450), Lactobacillus rhamnosus (for example CNCM 1-2449), Lactobacillus reuteri (for example CNCM 12451), Lactobacillus reuteri (for example CNCM I-2452), Lactobacillus acidophilus (for example CNCM 1-2453), Enterecoccus faecium (for example NCIMB 10415) and Bifidobacterium adolescentis.

A preferred probiotic strain useful for the present invention is Enterecoccus faecium NCIMB 10415, available under the Trademark CYLACTIN®.

Kaolin, also called china clay, is widely used in the making of paper, rubber, paint, and many other products. In its natural state kaolin is a white, soft powder consisting principally of the mineral kaolinite, which, under the electron microscope, is seen to consist of roughly hexagonal, platy crystals ranging in size from about 0.1 micrometer to 10 micrometers or even larger. Kaolin as found in nature usually contains varying amounts of other minerals such as muscovite, quartz, feldspar, and anatase. In addition, crude kaolin is frequently stained yellow by iron hydroxide pigments. It is often necessary to bleach the clay chemically to remove the iron pigment and to wash it with water to remove the other minerals in order to prepare kaolin for commercial use. In accordance with the present invention, the main function of kaolin is binding and absorbing.

The powder composition further contains a mixture of conventional additives, such as flavorings, sweeteners, additional absorbing & drying agents and fillers.

The mixture of conventional additives may contain at least one flavoring or sweetener agent, preferably a natural sweetener such as LUCTAROM®, powdery substances such as whole-wheat and silica powder, and at least one vegetable oil such as colza oil.

Whole-wheat (flour) is a powdery substance, a basic food ingredient, derived by grinding or mashing the whole grain of wheat. Whole-wheat flour is normally used in baking of breads and other baked goods, and also typically mixed with other lighter "white" unbleached or bleached flours (that have been treated with flour bleaching agent(s)) to restore nutrients to the white flours (especially fiber, protein, and vitamins), texture, and body that are lost in milling and other processing to the finished baked goods or other food(s).

Silica also known as Silicon dioxide is a mineral, which has - in accordance with the present invention - the main function of drying.

Colza oil is an oil obtained from the seeds of *Brassica napus.* In commerce, colza is a traditional rapeseed oil (with turnip rape oil, sarson oil, toria oil (*Brassica rapa* ssp). It is a comparatively non-odoriferous oil of a yellow color, having a specific gravity varying between 0.912 and 0.920.

Examples of particularly preferred dosages (by weight ratio) of the compounds in a final powder composition according to the invention are independently from each other in the following ranges:
CYLACTIN® between 0.5% and 1%, for example about 0.7%
LUCTAROM® between 0.1% and 0.5%, for example about 0.15%
TIXOSIL® (Silica powder) between 5% and 10%, for example about 6%
Kaolin between 50% and 70%, for example about 60%
Whole wheat between 20% and 40%, for example about 31%
Colza oil between 0.2% and 0.6%, for example about 0.5%

In a preferred embodiment of the invention the powder composition is intended to be used for newborn piglets. In this particular case, the composition is dispersed at the day of parturition at least once, for example at least twice, preferably three times on the floor of the farrowing (parturition) area and optionally onto the skin of the piglets and sows. In the following 2 to 10 days the number of applications can be reduced. Preferably the composition is applied at the day of parturition and at the following 2-3 days.

As discussed above, the present invention also provides methods for preventing diseases and enhancing the immune system of piglets.

In one aspect, the invention provides a method for preventing arthritis, neonatal diarrhea and skin disorders in newborn piglets by applying on the floor of the farrowing (parturition) area a powder composition comprising at least one probiotic lactic acid bacteria combined with kaolin and a mixture of conventional additives, such as flavorings, sweeteners, absorbing agents, drying agents and fillers, wherein the powder composition is applied starting from the day of parturition for a period of at least 2 - 4 days.

In another aspect the invention provides a composition for reducing temperature loss of newborn piglets during the first 1 - 4 days of life by applying on the floor of the farrowing (parturition) area a powder composition comprising at least one probiotic lactic acid bacteria combined with kaolin and a mixture of conventional additives, such as flavorings, sweeteners, absorbing agents, drying agents and fillers.

The present invention is described in detail below by a specific example. However, the present invention is not limited by that example.

### EXAMPLE: Hygienic, disinfectant powder composition for newborn piglets

Dosages of the compounds (by weight ratio) in the final powder composition are:

| | |
|---|---|
| CYLACTIN® ME20 Plus: | 0.77% |
| LUCTAROM®L 1336Z: | 0.15% |
| TIXOSIL®: | 6.16% |
| Kaolin: | 61.6% |
| Whole wheat: | 30.81% |
| Colza oil: | 0.46% |

For newborn piglets this specific powder composition is used at least during the first 4 days of life in amounts of approximately 50 - 70 g/litter/day.

## Claims

1. A hygienic powder composition comprising a lactic acid bacteria with probiotic properties and kaolin as absorbing and/or drying agent according for preventing diseases and enhancing the immune system of piglets, wherein the composition is dispersed at least once a day on the floor of the farrowing (parturition) area, wherein the composition is applied at the day of parturition and at least at the 2 following days and wherein the powder composition comprises by weight ratio 0.25% - 5% of a probiotic formulation containing at least one lactic acid bacteria and 40% - 70% kaolin.

2. A hygienic powder composition according to claim 1 for preventing neonatal diarrhea and skin disorders in newborn piglets, wherein the composition is dispersed at least once a day on the floor of the farrowing (parturition) area and wherein the composition is applied at the day of parturition and at least at the 2 following days.

3. A hygienic powder composition according to claim 1 to dry body surface of newborn piglets, wherein the composition is dispersed at least once a day on the floor of the farrowing (parturition) area and wherein the composition is applied at the day of parturition and at least at the 2 following days.

4. A hygienic powder composition according to claim 1 to reduce temperature loss of newborn piglets, wherein the composition is dispersed at least once a day on the floor of the farrowing (parturition) area and wherein the composition is applied at the day of parturition and at least at the 2 following days.

## Patentansprüche

1. Hygiene-Pulverzusammensetzung, umfassend ein Milchsäurebakterium mit probiotischen Eigenschaften und Kaolin als Absorptions- und/oder Trocknungsmittel zur Vorbeugung gegen Krankheiten und zur Stärkung des Immunsystems von Ferkeln, wobei die Zusammensetzung mindestens einmal täglich auf dem Boden des Abferkelbereichs (Geburtsbereichs) verteilt wird, wobei die Zusammensetzung am Tag der Geburt und mindestens an den 2 nachfolgenden Tagen angewandt wird und wobei die Pulverzusammensetzung, bezogen auf das Gewichtsverhältnis, 0,25% - 5% einer probiotischen Formulierung umfasst, die mindestens ein Milchsäurebakterium und 40% - 70% Kaolin enthält.

2. Hygiene-Pulverzusammensetzung nach Anspruch 1 zur Verhinderung von Neugeborenen-Durchfall und Hauterkrankungen bei neugeborenen Ferkeln, wobei die Zusammensetzung mindestens einmal täglich auf dem Boden des Abferkelbereichs (Geburtsbereichs) verteilt wird und wobei die Zusammensetzung am Tag der Geburt und mindestens an den 2 nachfolgenden Tagen angewandt wird.

3. Hygiene-Pulverzusammensetzung nach Anspruch 1 zum Trocknen der Körperoberfläche neugeborener Ferkel, wobei die Zusammensetzung mindestens einmal täglich auf dem Boden des Abferkelbereichs (Geburtsbereichs) verteilt wird und wobei die Zusammensetzung am Tag der Geburt und mindestens an den 2 nachfolgenden Tagen angewandt wird.

4. Hygiene-Pulverzusammensetzung nach Anspruch 1 zur Verringerung des Temperaturverlusts von neugeborenen Ferkeln, wobei die Zusammensetzung mindestens einmal täglich auf dem Boden des Abferkelbereichs (Geburtsbereichs) verteilt wird und wobei die Zusammensetzung am Tag der Geburt und mindestens an den 2 nachfolgenden Tagen angewandt wird.

## Revendications

1. Composition de poudre hygiénique comprenant une bactérie d'acide lactique dotée de propriétés probiotiques et du kaolin en tant qu'agent absorbant et/ou séchant pour la prévention de maladies et l'augmentation du système immunitaire de porcelets, la composition étant dispersée au moins une fois par jour sur le sol de la zone de mise bas (parturition), la composition étant appliquée le jour de la parturition et au moins les 2 jours suivants et la composition de poudre comprenant en rapport en poids 0,25 % à 5 % d'une formulation probiotique contenant au moins une bactérie d'acide lactique et 40 % à 70 % de kaolin.

2. Composition de poudre hygiénique selon la revendication 1 pour la prévention de la diarrhée néonatale et de troubles cutanés chez des porcelets nouveau-nés, la composition étant dispersée au moins une fois par jour sur le sol de la zone de mise bas (parturition) et la composition étant appliquée le jour de la parturition et au moins les 2 jours suivants.

3. Composition de poudre hygiénique selon la revendication 1 pour sécher la surface corporelle de porcelets nouveau-nés, la composition étant dispersée au moins une fois par jour sur le sol de la zone de mise bas (parturition) et la composition étant appliquée le jour de la parturition et au moins les 2 jours suivants.

4. Composition de poudre hygiénique selon la revendication 1 pour réduire la perte de température de porcelets nouveau-nés, la composition étant dispersée au moins une fois par jour sur le sol de la zone de mise bas (parturition) et la composition étant appliquée le jour de la parturition et au moins les 2 jours suivants.
